# EUROPEAN PATENT SPECIFICATION

(11) **EP 1 319 387 B1**
(45) Date of publication and mention of the grant of the patent: **23.04.2008**
(21) Application number: 02291541.7
(22) Date of filing: 20.06.2002
(51) Int. Cl.: A61Q 1/12

(54) **Cosmetic compositions of powder-type containing whitening ingredient**
Kosmetische Zusammensetzungen in Form eines Pulvers enthaltend Hautweissungsmittel
Composition cosmetique sous la forme d'une poudre comprenant des agents de blanchiment epidermiques

(30) Priority: 14.12.2001 KR 2001079249
(43) Date of publication of application: 18.06.2003
(73) Proprietor: Coreana Cosmetics Co., Ltd., Cheonan-si, Chungcheongnam-do (KR)
(72) Inventor: Kim, Dong-Myung, Paldal-Gu, Suwon-Si, Kyoungki-Do (KR); Jo, Byoung-Kee, Dongan-Gu, Anyang-Si, Kyoungki-Do (KR); Kim, Hyo-Yong, Kwangju-Si (KR)
(74) Representative: Kedinger, Jean-Paul

(56) References cited:
- EP-A- 0 436 729
- EP-A- 1 206 928
- US-A- 5 411 741

## Description

### BACKGROUND OF THE INVENTION

### Field of the Invention

The present invention relates to powder-type cosmetic compositions which contain a stabilized whitening ingredient with an excellent whitening effect, and more particularly to powder-type cosmetic compositions having little skin irritant effect and excellent skin whitening effect, in the form of a powder containing a large amount of liquid phase ingredient.

### Description of the Related Art

During childhood, the skin rapidly recovers from damage caused by sunlight. But, as we get older, wrinkles, calluses, sagging, inelasticity, roughness, dryness, mottling, etc., of the skin by exposure to sunlight are likely to happen. The general term for these changes is "photo aging". Namely, photo aging damages the epidermal tissue and hypodermal tissue of the skin. Also, it causes wrinkles to form, and skin to turn yellowish, thick and inelastic.

Recent industrial and economic development has led to increased numbers of vehicles such as automobiles, and household appliances such as heaters and air-conditioners. As such, air pollution and noise pollution are growing more and more serious. Pollutants from automobiles, particularly nitrogen compounds, contain ozone-depleting material and thus destroy the ozone layer. In addition to these pollutants, water pollution and soil pollution are increasing sharply. Also, allergenic sensitization to pollen in spring and fall is a cause of contact dermatitis.

Many skin care cosmetics to protect the skin against photo aging and various pollutants as mentioned above have been developed. Cosmetics for reducing wrinkles, cosmetics for skin whitening and cosmetics for protection from the sun can be exemplified.

Examples of cosmetic formulations which can impart moisturizing, anti-wrinkling or whitening effect to the skin are typical skin care formulations such as after shave, lotions and creams, and makeup formulations for beautifying the skin by coloring the skin and for masking skin troubles such as melasma and ephelides. Among these, powder-type cosmetic formulations containing liquid phase ingredients such as anti-wrinkling or whitening ingredients are known.

Japanese Patent Laid-Open Nos. 6-166611 and 5-65212 describe powder-type cosmetic compositions containing a large amount of liquid phase ingredient, comprising a hydrophobized silica having specific surface area of 80 m²/g or more, a powder surface-treated with a fluorine compound, an oily ingredient and an aqueous ingredient (30-90%). These compositions can absorb water by their containing amorphous silica. But, on account of the high hydrophilicity of the powder surface, formulation into powder is very difficult. So, the amorphous silica is appropriately hydrophobized by a silicone compound and a fluorine compound in these applications.

However, the formulations have disadvantages that they are difficult to formulate homogeneously when the liquid phase ingredient is added in a heterogeneous mixing state with the powder or the mixing speed of a mixer is not constant. Also, the powders tend to aggregate during the production of the cosmetic compositions. Particularly, it is difficult to mix the powder with a whitening ingredient such as licorice extract, vitamin C and its derivatives, etc., and titer and stability of the compositions are not maintained for a long time.

On the other hand, examples of cosmetic compositions produced by stabilizing a whitening ingredient, e.g., vitamin C and its derivatives, include emulsion-type cosmetics such as oil in water (O/W) type and water in oil (W/O) type.

But, in the emulsion-type cosmetics, since water is highly compatible with the powder containing a large amount of the liquid phase ingredient, the powder readily coagulates. As a result, there are a number of disadvantages that they are difficult to formulate into a powder and are very tacky upon use. Particularly, titer retention is reduced, and discoloration and malodor are likely to develop, so that liquid phase-containing powder is not suitable for formulation as emulsion type cosmetics.

### SUMMARY OF THE INVENTION

Therefore, the present invention has been made in view of the above-mentioned problems, and it is an object of the present invention to provide powder-type cosmetic compositions containing a large amount of liquid phase ingredient, which can impart whitening effect to the skin, mask skin troubles, stabilize a whitening ingredient and be easily penetrated into the skin.

### DETAILED DESCRIPTION OF THE PREFERRED EMBODIMENT

The present invention relates to liquid phase ingredient-containing powder-type cosmetic compositions which comprise: a whitening ingredient-containing powder produced by dissolving a whitening ingredient in a solvent, encapsulating the solution by a crosslinked acryl powder, and surface-treating the capsule using a hydrophobic polymer; a mixed powder including one or more powders selected from the group consisting of polymethylsilsesquioxane having an average particle size of 3.5 µm and a specific surface area of 110-150 m²/g, HDI/Trimethylol hexyllatactone and silica cross polymer having an average particle size of 3.7 µm and a specific surface area of 150-180 m²/g, laurylmethacrylate/glycoldimethacrylate cross polymer having an average particle size of 25 µm and a specific surface area of 100-300 m²/g and an amorphous silica having an average particle size of 5.0 µm and an oil absorption of 0.3-4 ml/g; and a polyol/silicone emulsifying base for stabilizing a formulation and the whitening ingredient.

Specifically, the powder-type cosmetic compositions according to the present invention are produced by the steps of dissolving a whitening ingredient in a solvent, encapsulating the solution with a crosslinked acryl powder, coating the capsule with a hydrophobic polymer, and finally applying the coated powder into a powder containing a large amount of liquid phase ingredient.

The present inventor has continuously and intensively studied the powder-type cosmetic compositions which can impart whitening effect to the skin, mask skin troubles, stabilize whitening ingredient and easily penetrate into the skin, and as a result, has developed a powder containing a large amount of liquid phase ingredient using an amorphous powder capable of effectively absorbing the liquid phase ingredient. Further, the present inventor produced a whitening ingredient-containing powder by dissolving a whitening ingredient such as vitamin C and its derivatives, kojic acid, mulberry extract, in a solvent such as ester or butylene, encapsulating the solution with a crosslinked acryl powder, and coating the capsule with a hydrophobic polymer to stabilize the powder. The whitening ingredient-containing powder thus produced above releases the whitening ingredient slowly during the use of the cosmetic compositions containing such powder.

The cosmetic compositions of the present invention contain 1.0-10.0% by weight of the whitening ingredient.

According to a preferred embodiment of the invention, there is provided a powder in which the whitening ingredient is stabilized and its titer is well preserved.

First, a material effective for whitening such as arbutin, kojic acid, vitamin C and its derivatives, licorice extract, mulberry extract, ethylascorbyl ether, magnesium ascorbyl phosphate, and at least one antioxidant selected from the group consisting of BHT[2,6 bis(1,1-dimethylethyl)-4-methoxyphenol], BHA[butylated hydroxyanisole], tocopheryl acetate, polyphenol and camphor in an amount of 1-30% by weight of the encapsulated powder to be produced are dissolved in an oily or an aqueous solvent. Subsequently, the solution is encapsulated with a crosslinked acryl powder and then lyophilized to control the particle size of the capsule, depending on formulation properties.

Examples of the aqueous solvents which may be used in the invention include 1,3-butyleneglycol, glycerin, konjak . Examples of the oily solvents which may be used in the invention include hexylaurate, squalane, trioctyldodecylcitrate, etc. And all kinds of oily materials such as vegetable oils, animal oils, synthetic oils, mineral oils, can be used, provided that their liquid states can be maintained at room temperature.

For producing the whitening ingredient-containing capsule having excellent usability and stability in the final cosmetic compositions to be produced, the surface of the capsule is hydrophobically treated. When the hydrophobicity of the capsule is poor, the formulation into powder is difficult. When the hydrophobicity of the capsule is high, the usability of the final cosmetic compositions to be produced is very poor and the formulation is also very difficult to carry out. Therefore, the hydrophobic treatment of the capsule is a highly important process in the present invention.

Examples of the hydrophobizing compounds which are commonly used in the prior art include silicone-based compounds such as methicone, dimethicone, cyclomethicone, phenyltrimethicone, etc., fluorine compounds, amino acid salts, etc. When the capsule surface is treated using these compounds, its hydrophobicity is satisfactory but its stability and usability in the final cosmetic compositions to be produced are poor.

To overcome the above problems, a mixture of cyclomethicone, dimethicone, vinyl dimethicone cross polymer and cetyl dimethicone copolyol is used for the hydrophobic surface treatment of the capsule in the present invention.

When surface-treating the capsule, an optimal content of the hydrophobizing compounds is from 0.5 to 5% by weight, preferably from 0.5 to 2.0% by weight of the encapsulated powder.

When the content is less than 0.5% by weight, the hydrophobicity is poor so that the whitening ingredient is released into the aqueous solvent and so the usability of the final cosmetic compositions becomes poor. When the content is more than 2.0% by weight, the stability of the capsule is high but the affinity for water is little or none so that the formulation becomes difficult and the usability of the final cosmetic compositions to be produced is poor.

The relative weight ratio of the silicone-based compounds, i.e. cyclomethicone, dimethicone, vinyl dimethicone cross polymer and cetyl dimethicone copolyol is 20-40: 30-60: 3-10: 0.1-2.0, based on the total weight of the hydrophobizing compounds.

According to another preferred embodiment of the invention, there is provided a powder containing a large amount of liquid phase ingredient.

In order to effectively absorb water, a mixed powder including one or more powders selected from the group consisting of polymethylsilsesquioxane having an average particle size of 3.5 µm and a specific surface area of 110-150 m²/g, HDI/Trimethylol hexyllatactone and silica cross polymer having an average particle size of 3.7 µm and a specific surface area of 150-180 m²/g, laurylmethacrylate/glycoldimethacrylate having an average particle size of 25 µm and a specific surface area of 100-300 m²/g, and an amorphous silica having an average particle size of 5.0 µm is used.

The mixed powder can be used in an amount ranging from 5 to 50% by weight, based on the total weight of the final cosmetic compositions to be produced.

In order to impart other colors to the powder, iron oxides, titanium dioxide or pearl of an appropriate amount can be additionally blended.

According to a third preferred embodiment of the invention, there is provided a powder containing a large amount of liquid phase ingredient and the stabilized whitening ingredient.

When the powder is mixed with the liquid phase ingredient, coagulation occurs and their compatibility decreases.

To solve these problems, polyol (aqueous ingredient)/silicone emulsifying base is used in the present invention. The emulsifying base enables the formulation to be easily made and stabilize the whitening ingredient. Also, the emulsifying base makes the initial dispersion of the powder easy and enhances adhesiveness and durability of the aqueous ingredient when applied to the skin. Further, the emulsifying base prevents the powder from being dispersed by air currents.

Examples of the polyol which may be employed in the emulsifying base include glycerin propylene glycol, mannan, soyful, etc., and examples of the silicone include cyclomethicone, dimethicone, vinyl dimethicone silicone powder, cetyl dimethicone copolyol, etc.

The polyol and silicone can be present in an amount of from 5 to 20% and from 2 to 10% by weight of the total weight of the emulsifying base, respectively.

Purified water can be added to the emulsifying base in an amount of from 70 to 93% by weight of the total weight of the emulsifying base. In the end, the liquid phase ingredient such as water, glycerin, etc., is present in an amount of from 50 to 80% by weight, based on the final powder-type cosmetic compositions.

The following examples are provided for purposes of illustration only and are not intended to limit the present invention.

### [Preparative Example]

First, a capsule was produced by dissolving whitening ingredients, vitamin C and the other effective ingredients of the following % by weight listed in Table 1 in an oily medium, and then encapsulating the solution with a crosslinked acryl powder.

**[Table 1]**

| No | Ingredients | Sample 1 | Sample 2 |
|---|---|---|---|
| 1 | Crosslinked acryl powder | 83.7 | 81.7 |
| 2 | Vitamin C | - | 3.0 |
| 3 | 1,3-butylene glycol | - | 15.0 |
| 4 | Licorice extract | 1.0 | - |
| 5 | Hexylaurate | 15.0 | - |
| 6 | BHT | 0.2 | 0.2 |
| 7 | Methyl paraffin | 0.1 | 0.1 |

### (Procedure)

1) Ingredient 1 was charged into a high speed Henshel mixer, and mixed at 1000 rpm.
2) Ingredients 2 to 7 were dissolved using a high speed Ultra mixer at room temperature.
3) The mixture obtained in 1) above was slowly added while mixing the solution obtained in 2) above at 3000 rpm, and then lyophilized.

Next, the capsule obtained thus was hydrophobically surface-treated with the ingredients of the following % by weight listed in Table 2.

**[Table 2]**

| No. | Ingredients | Sample 3 | Sample 4 |
|---|---|---|---|
| 1 | Sample 1 | 98 | - |
| 2 | Sample 2 | - | 97.5 |
| 3 | Cyclomethicone | 0.50 | 0.40 |
| 4 | Dimethicone | 1.20 | 1.33 |
| 5 | Vinyl dimethicone cross polymer | 0.20 | 0.20 |
| 6 | Cetyl dimethicone copolyol | 0.10 | 0.07 |

### (Procedure)

1) Ingredients 3-6 were mixed using a high speed Ultra mixer at 3500 rpm for 20 minutes.
2) Ingredients 1 and 2 were charged into a high speed Henshel mixer and mixed at 1000 rpm. Ingredient 1 was sprayed thereon and then lyophilized.

A polyol/silicone emulsifying base was produced in accordance with the ingredients of the following % by weight listed in Table 3.

**[Table 3]**

| No. | Ingredients | Sample 5 | Sample 6 |
|---|---|---|---|
| 1 | Cetyl dimethicone copolyol | 0.4 | 0.5 |
| 2 | Dimethicone (6 cs) | 10.0 | 5.0 |
| 3 | Dimethicone (8 cs) | - | 5.0 |
| 4 | Cyclomethicone | 3.0 | 5.0 |
| 5 | Lauryl dimethicone copolyol | 0.3 | 0.5 |
| 6 | Purified water | To 100 | To 100 |
| 7 | Glycerin | 10.0 | 5.0 |
| 8 | 1,3-butylene glycol | 5.0 | 10.0 |
| 9 | Konjak | 0.5 | 0.5 |
| 10 | D,L-panthenol | 0.1 | 0.1 |
| 11 | Preservative | q.s | q.s |
| 12 | Flavoring agent | q.s | q.s |

### (Procedure)

Ingredients 1-5 were heated to 70-75°C to form an oily phase. Ingredients 6-11 were heated to 70-75°C and ingredient 11 was added thereto to form an aqueous phase. The aqueous phase was slowly added to the oily phase, and then emulsified using Homo Rpm 3000 for 10 minutes. Ingredient 12 was added to the emulsion, emulsified for 1 minute and then cooled to 30°C.

Finally, the powder-type cosmetic compositions according to the present invention were prepared with ingredients of the following % by weight listed in Table 4, including the whitening ingredients-containing powder and the emulsifying base produced above.

**[Table 4]**

| No. | Ingredients | Exam. 1 | Exam. 2 | Exam. 3 | Comp. Exam 1 | Comp. Exam 2 |
|---|---|---|---|---|---|---|
| 1 | Sample 1 | 3.0 | - | - | - | - |
| 2 | Sample 3 | - | 3.0 | - | - | - |
| 3 | polymethylsilsesquioxane | 5.0 | 3.0 | 5.0 | 5.0 | - |
| 4 | Licorice extract | - | - | - | - | 0.5 |
| | (Squalane 0.10%) | | | | | |
| 5 | HDI/Trimethylol | 5.0 | 12.0 | 5.0 | 5.0 | - |
| | hexyllatactone/silica | | | | | |
| | cross polymer (and) | | | | | |
| | Silica | | | | | |
| 6 | Silica (hydrophobic) | - | - | - | - | 10.0 |
| 7 | Licorice extract | - | - | - | 3.0 | 3.0 |
| 8 | Purified water | - | - | - | To 100 | To 100 |
| 9 | Condensed glycerin | - | - | - | 8.3 | - |
| 10 | 1,3-butylen glycol | - | - | - | 4.15 | - |
| 11 | Preservative | - | - | - | q.s | q.s |
| 12 | Sample 5 | To | - | To | - | - |
| | | 100 | | 100 | | |
| 13 | Sample 6 | - | To | - | - | - |
| | | | 100 | | | |
| 14 | Sample 2 | - | - | 3.0 | - | - |
| 15 | Licorice extract | - | - | - | - | 0.5 |
| | (O/W emulsion 0.1%) | | | | | |
| 16 | Polyphenol | 0.2 | 0.2 | 0.2 | 0.2 | 0.2 |

### (Procedure)

1) Ingredients 1-6 were charged into a Henshel mixer, and mixed.
2) Ingredients 7-16 were homogeneously mixed using AGI mixer, and sprayed onto the mixture obtained in 1) above to obtain a formulation.

### [Experimental Example 1]

The physical state of the formulations produced in the above Examples and Comparative Examples was evaluated by a sensory test. The stability of the compositions was evaluated in terms of discoloration/malodor and titer retention. The physical state of the powder was evaluated by the naked eye, and the results presented in the following Table 5. The discoloration/malodor tests were performed after storing the compositions at 45°C for 4 weeks, and titer retention test was performed by measuring the absorbance using an HPLC immediately after their productions and after storing the compositions at 45°C for 1 month, respectively. The results are listed in the following Table 6.

**[Table 5]**

| | Exam. 1 | Exam. 2 | Exam. 3 | Comp. Exam. 1 | Comp. Exam. 2 |
|---|---|---|---|---|---|
| 0 day | a | a | b | c | c |
| 1 month (45°C) | c | b | a | d | d |

| | | | | | |
|---|---|---|---|---|---|
| a: ≥ 95% excellent, b: ≥ 80% good, c: ≥ 70% average, d: < 50% poor | | | | | |

**[Table 6]**

| | Exam. 1 | | Exam. 2 | | Exam. 3 | | Comp. Exam. 1 | | Comp. Exam. 2 | |
|---|---|---|---|---|---|---|---|---|---|---|
| Discoloration /malodor (45°C, 4weeks) | c | | b | | a | | d | | d | |
| Titer retention (%) | 0 day | 1 mon | 0 day | 1 mon | 0 day | 1 mon | 0 day | 1 mon | 0 day | 1 mon |
| | 99.4 | 99.0 | 99.7 | 98.6 | 99.5 | 97.3 | 98.4 | 55.5 | 98.6 | 57.6 |

| | | | | | | | | | | |
|---|---|---|---|---|---|---|---|---|---|---|
| a: ≥ 95% excellent, b: ≥ 80% good, c: ≥ 70% average, d: < 50% poor. | | | | | | | | | | |

It has been proven from the Tables 5 and 6 that the compositions according to the present invention can be more feasibly formulated into powder and stored longer than the Comparative Examples. It is assumed that the coagulation of powder is inhibited and stability at high temperatures is excellent by treating the surface of the powder with silicone mixture and using polyol/silicone emulsifying base.

Also, the compositions of the Examples were superior to those of the Comparative Examples in terms of titer retention after storing the compositions at 45°C for 1 month. This result indicats that the emulsified films of the powder are more stable, due to treating the surface of the powder with silicone mixture and using polyol/silicone emulsifying base.

### [Experimental Example 2]

The powder-type cosmetic compositions according to the present invention were evaluated by measuring the skin irritations and liquefied states upon their use.

The measurement of skin irritations was carried out in a sensory test by adhering patches to 20 healthy women aged 25-35 and then removing the patches after 24 hours. When no irritation to the skin was not observed, the composition was scored as "0". Severe irritaion was scored as "5", and the scores are expressed as averages. The results are listed in the following Table 7. Also, after the powder-type cosmetic compositions of the Examples and Comparative Examples were applied to the test subjects once a day for 1 month, the sensory tests for usability and liquefied state upon use of the compositions were carried out. The results are listed in the following Table 8.

**[Table 7]**

| | Exam. 1 | Exam. 2 | Exam. 3 | Comp. Exam. 1 | Comp. Exam. 2 |
|---|---|---|---|---|---|
| Skin irritation | 0.4 | 0.7 | 0.9 | 3.3 | 2.7 |

**[Table 8]**

| | Exam. 1 | Exam. 2 | Exam. 3 | Comp. Exam. 1 | Comp. Exam. 2 |
|---|---|---|---|---|---|
| Usability | a | b | a | d | e |
| Liquefied state | b | b | a | c | c |

| | | | | | |
|---|---|---|---|---|---|
| a: excellent, b: 80% good, c: 70% average, d: below average, e: poor | | | | | |

As can be seen from the Table 7, the compositions of the Examples exhibited superior stability to those of the Comparative Examples. From this observation, it is assumed that the whitening ingredient, e.g., licorice extract, encapsulated in the capsule is slowly released to minimize skin irritation.

As can be seen from the Table 8, the compositions of Examples exhibited more excellent usability and liquefied state upon use than those of Comparative Examples. This result shows that since powder-type cosmetic compositions of the present invention contain the liquid phase ingredient and polyol/silicone emulsifying base, they have a pleasant feel upon use in spite of containing powder.

### [Experimental Example 3]

In order to evaluate the whitening effect of the powder-type cosmetic compositions according to the present invention, the color change of the cosmetic compositions of Examples and Comparative Examples produced in accordance with the above Table 4 was tested.

Two compositions selected from the cosmetic compositions of Examples and Comparative Examples were respectively applied on each of both sides of the faces of 20 women aged 25-35 as test subjects twice a day for 2 months, under indoor and outdoor environmental conditions of a temperature of 24-26°C and at a relative humidity of 38-40%. After 2 months, the color change was evaluated by measuring the lightness (L) of face by means of a colorimeter before and after the application. The differences of L values measured between the right and left side faces are represented as □E in the following Table 9.

**[Table 9]**

| | Exam. 1 | Exam. 2 | Exam. 3 | Comp. Exam. 1 | Comp. Exam. 2 |
|---|---|---|---|---|---|
| □E | 1.0 | 1.4 | 1.5 | 0.20 | 0.35 |

As can be seen from the Table 9, the compositions of Examples exhibited superior whitening effect to the Comparative Examples. This result shows that the encapsulated whitening ingredient effectively penetrates into the skin.

As described above, the powder-type cosmetic compositions according to the present invention can impart whitening effect to the skin, mask skin troubles, stabilize whitening ingredients and easily penetrate into the skin. At this time, the whitening ingredient encapsulated in the capsule is slowly released to minimize skin irritation.

Further, the cosmetic compositions according to the present invention can maintain their titer for a long time and prevent the powder from being dispersed by air currents.

## Claims

1. A powder-type cosmetic composition comprising:
a whitening ingredient-containing powder produced by dissolving a whitening ingredient and an antioxidant in a solvent, encapsulating the solution by a crosslinked acryl powder, and surface-treating the capsule with a hydrophobic polymer;
a mixed powder including one or more powders selected from the group consisting of polymethylsilsesquioxane having an average particle size of 3.5 µm and a specific surface area of 110-150 m²/g, HDI/Trimethylol hexyllatactone and silica cross polymer having an average particle size of 3.7 µm and a specific surface area of 150-180 m²/g, laurylmethacrylate/glycoldimethacrylate cross polymer having an average particle size of 25 µm and a specific surface area of 100-300 m²/g and an amorphous silica having an average particle size of 5.0 µm and an oil absorption of 0.3-4 ml/g; and
a polyol/silicone emulsifying base.

2. The cosmetic composition as set forth in claim 1, wherein the whitening ingredient is at least one selected from the group consisting of arbutin, kojic acid, vitamin C and its derivatives, licorice extract, mulberry extract, ethylascorbyl ether and magnesium ascorbyl phosphate.

3. The cosmetic composition as set forth in claim 1 or 2, wherein the whitening ingredient is present in an amount 1.0-10.0% by weight of the final cosmetic composition.

4. The cosmetic composition as set forth in claim 1, wherein the solvent is an aqueous solvent selected from 1,3-butyleneglycol, glycerin and konjak, or an oily solvent selected from hexylaurate, squalane, trioctyldodecylcitrate, vegetable oils, animal oils, synthetic oils and mineral oils.

5. The cosmetic composition as set forth in claim 1, wherein the antioxidant is selected from the group consisting of BHT[2,6 bis(1,1-dimethylethyl)-4-methoxyphenol], BHA[butylated hydroxyanisole], tocopheryl acetate, polyphenol and camphor, and is present in an amount ranging from 0.05 to 2.0% by weight of total weight of the final cosmetic composition.

6. The cosmetic composition as set forth in claim 1, wherein the hydrophobic polymer is a mixture of at least one polymer selected from cyclomethicone, dimethicone, vinyl dimethicone cross polymer and cetyl dimethicone copolyol.

7. The cosmetic composition as set forth in claim 1 or 6, wherein the hydrophobic polymer is present in an amount of from 0.5 to 5% by weight of the encapsulated powder.

8. The cosmetic composition as set forth in claim 1 or 6, wherein the relative weight ratio of cyclomethicone, dimethicone, vinyl dimethicone cross polymer and cetyl dimethicone copolyol is 20-40: 30-60: 3-10: 0.1-2.0, based on the total weight thereof.

9. The cosmetic composition as set forth in claim 1, wherein the mixed powder is present in an amount ranging from 5 to 50% by weight of the total weight of the final cosmetic composition.

10. The cosmetic composition as set forth in claim 1, wherein the polyol of the polyol/silicone emulsifying base is selected from glycerin propylene glycol, mannan and soyful, and the silicone of the polyol/silicone emulsifying base is selected from cyclomethicone, dimethicone, vinyl dimethicone silicone powder and cetyl dimethicone copolyol.

11. The cosmetic composition as set forth in claim 1 or 10, wherein the polyol and silicone of the polyol/silicone emulsifying base is present in an amount of from 5 to 20% and from 2 to 10% by weight of the total weight of the emulsifying base, respectively, and purified water is added to the emulsifying base in an amount of from 70 to 93% by weight of the total weight of the emulsifying base.

12. The cosmetic composition as set forth in claim 1, wherein liquid phase ingredient is present in an amount of from 50 to 80% by weight, based on the final cosmetic composition.

## Patentansprüche

1. Pulverartige kosmetische Zusammensetzung, umfassend:
ein einen Weißungsbestandteil enthaltendes Pulver, das durch Lösen eines Weißungsbestandteils und eines Antioxidationsmittels in einem Lösungsmittel, Verkapseln der Lösung mithilfe eines vernetzten Acrylpulvers und Oberflächenbehandlung der Kapsel mit einem hydrophoben Polymer hergestellt wurde;
ein Pulvergemisch mit einem oder mehreren Pulvern, ausgewählt aus der Gruppe, bestehend aus Polymethylsilsesquioxan mit einer durchschnittlichen Teilchengröße von 3,5 µm und einer spezifischen Oberfläche von 110-150 m²/g, einem vernetzten Polymer aus HDI/Trimethylolhexyllatacton und Siliciumdioxid mit einer durchschnittlichen Teilchengröße von 3,7 µm und einer spezifischen Oberfläche von 150-180 m²/g, einem vernetzten Polymer aus Laurylmethacrylat/Glycoldimethacrylat mit einer durchschnittlichen Teilchengröße von 25 µm und einer spezifischen Oberfläche von 100-300 m²/g und einem amorphen Siliciumdioxid mit einer durchschnittlichen Teilchengröße von 5,0 µm und einer Ölabsorption von 0,3-4 ml/g; und
einer Polyol/Silikon-Emulgierbasis.

2. Kosmetische Zusammensetzung nach Anspruch 1, wobei der Weißungsbestandteil mindestens einer ausgewählt aus der Gruppe, bestehend aus Arbutin, Kojinsäure, Vitamin C und dessen Derivate, Lakritzextrakt, Maulbeerextrakt, Ethylascorbylether und Magnesiumascorbylphosphat, ist.

3. Kosmetische Zusammensetzung nach Anspruch 1 oder 2, wobei der Weißungsbestandteil in einer Menge von 1,0-10,0 Gew.-% der fertigen kosmetischen Zusammensetzung vorliegt.

4. Kosmetische Zusammensetzung nach Anspruch 1, wobei das Lösungsmittel ein wässriges Lösungsmittel, ausgewählt aus 1,3-Butylenglycol, Glycerin und Konjak, oder ein öliges Lösungsmittel, ausgewählt aus Hexyllaurat, Squalan, Trioctyldodecylcitrat, Pflanzenölen, tierischen Ölen, synthetischen Ölen und Mineralölen, ist.

5. Kosmetische Zusammensetzung nach Anspruch 1, wobei das Antioxidationsmittel ausgewählt ist aus der Gruppe, bestehend aus BHT [2,6-Bis(1,1-dimethylethyl)-4-methoxyphenol], BHA [butyliertes Hydroxyanisol], Tocopherylacetat, Polyphenol und Kampfer, und in einer Menge im Bereich von 0,05 bis 2,0 Gew.-% des Gesamtgewichts der fertigen kosmetischen Zusammensetzung vorliegt.

6. Kosmetische Zusammensetzung nach Anspruch 1, wobei das hydrophobe Polymer eine Mischung aus mindestens einem Polymer ist, ausgewählt aus Cyclomethicon, Dimethicon, vernetztem Vinyldimethicon-Polymer und Cetyldimethicon-Copolyol.

7. Kosmetische Zusammensetzung nach Anspruch 1 oder 6, wobei das hydrophobe Polymer in einer Menge von 0,5 bis 5 Gew.-% des verkapselten Pulvers vorliegt.

8. Kosmetische Zusammensetzung nach Anspruch 1 oder 6, wobei das relative Gewichtsverhältnis von Cyclomethicon, Dimethicon, vernetztem Vinyldimethicon-Polymer und Cetyldimethicon-Copolyol 20-40:30-60:3-10:0,1-2,0, bezogen auf das Gesamtgewicht davon, beträgt.

9. Kosmetische Zusammensetzung nach Anspruch 1, wobei das Pulvergemisch in einer Menge im Bereich von 5 bis 50 Gew.-% des Gesamtgewichts der fertigen kosmetischen Zusammensetzung vorliegt.

10. Kosmetische Zusammensetzung nach Anspruch 1, wobei das Polyol der Polyol/Silikon-Emulgierbasis ausgewählt ist aus Glycerinpropylenglycol, Mannan und Soyful, und das Silikon der Polyol/Silikon-Emulgierbasis ausgewählt ist aus Cyclomethicon, Dimethicon, Vinyldimethicon-Silikonpulver und Cetyldimethicon-Copolyol.

11. Kosmetische Zusammensetzung nach Anspruch 1 oder 10, wobei das Polyol und das Silikon der Polyol/Silikon-Emulgierbasis in einer Menge von 5 bis 20 Gew.-% bzw. von 2 bis 10 Gew.-% des Gesamtgewichts der Emulgierbasis vorliegen und gereinigtes Wasser in einer Menge von 70 bis 93 Gew.-% des Gesamtgewichts der Emulgierbasis zur Emulgierbasis gegeben wird.

12. Kosmetische Zusammensetzung nach Anspruch 1, wobei der Flüssigphasenbestandteil in einer Menge von 50 bis 80 Gew.-%, bezogen auf die fertige kosmetische Zusammensetzung, vorliegt.

## Revendications

1. Composition cosmétique sous forme de poudre comprenant:
une poudre contenant un ingrédient de blanchiment produite par la dissolution d'un ingrédient de blanchiment et d'un antioxydant dans un solvant, l'encapsulation de la solution à l'aide d'une poudre acrylique réticulée et le traitement de surface de la capsule avec un polymère hydrophobe;
une moudre mixte comprenant une ou plusieurs poudres choisies dans le groupe constitué par un polyméthylsilsesquioxane ayant une taille moyenne de particules de 3,5 µm et une aire de surface spécifique de 110-150 m²/g, un polymère réticulé de silice et de HDI/Triméthylol hexyllatactone ayant une taille moyenne de particules de 3,7 µm et une aire de surface spécifique de 150-180 m²/g, un polymère réticulé de méthacrylate de lauryle et de diméthacrylate de glycol ayant une taille moyenne de particules de 25 µm et une aire de surface spécifique de 100-300 m²/g et une silice amorphe ayant une taille moyenne de particules de 5,0 µm et une absorption d'huile de 0,3-4 ml/g; et
une base émulsifiante de polyol/silicone.

2. Composition cosmétique telle que définie dans la revendication 1, dans laquelle l'ingrédient de blanchiment est l'un au moins des éléments choisis dans le groupe constitué par l'arbutine, l'acide kojique, la vitamine C et ses dérivés, un extrait de réglisse, un extrait de mûrier, l'éthylascorbyl éther et l'ascorbyl phosphate de magnésium.

3. Composition cosmétique telle que définie dans la revendication 1 ou 2, dans laquelle l'ingrédient de blanchiment est présent en une quantité de 1,0-10,0 % en poids de la composition cosmétique finale.

4. Composition cosmétique telle que définie dans la revendication 1, dans laquelle le solvant est un solvant aqueux choisi entre 1,3-butylèneglycol, la glycérine et le konjac, ou un solvant huileux choisi entre le laurate d'hexylc, le squalanc, le citrate de trioctyldodécyle, des huiles végétales, des huiles animales, des huiles synthétiques et des huiles minérales.

5. Composition cosmétique telle que définie dans la revendication 1, dans laquelle l'antioxydant est choisi dans le groupe constitué par le BAHT[2,6 bis(1,1-diméthyléthyl)-4-méthoxyphénol], le BHA[hydroxyanisole butylaté], l'acétate de tocophéryle, un polyphénol et le camphre, et est présent en une quantité se situant dans une plage de 0,05 à 2 % en poids du poids total de la composition cosmétique finale.

6. Composition cosmétique telle que définie dans la revendication 1, dans laquelle le polymère hydrophobe est un mélange d'au moins un polymère choisi enture la cyclométhicone, la diméthicone, un polymère réticulé de vinyl diméthicone et un cétyl diméthicone copolyol.

7. Composition cosmétique telle que définie dans la revendication 1 ou 6, dans laquelle le polymère hydrophobe est présent en une quantité de 0,5 à 5 % en poids de la poudre encapsulée.

8. Composition cosmétique telle que définie dans la revendication 1 ou 6, dans laquelle le rapport de poids relatif de la cyclométhicone, de la diméthicone, du polymère réticulé de vinyl diméthicone et du cétyl diméthicone copolyol est 20-40: 30-60: 3-10: 0,1-2,0, par rapport à leur poids total.

9. Composition cosmétique telle que définie dans la revendication 1, dans laquelle la poudre mixte est présente en une quantité se situant dans une plage de 5 à 50 % en poids du poids total de la composition cosmétique finale.

10. Composition cosmétique telle que définie dans la revendication 1, dans laquelle le polyol de la base émulsifiante polyol/silicone est choisi entre le glycérine propylène glycol, le mannane et un extrait de soja (soyful), et la silicone de la base émulsifiante polyol/silicone est choisie entre la cyclométhicone, la diméthicone, une poudre de vinyl diméthicone silicone et un cétyl diméthicone copolyol.

11. Composition cosmétique telle que définie dans la revendication 1 ou 10, dans laquelle le polyol et la silicone de la base émulsifiante polyol/silicone sont respectivement présents en une quantité de 5 à 20 % et de 2 à 10 % en poids du poids total de la base émulsifiante, et de l'eau purifiée est ajoutée à la base émulsifiante en une quantité de 70 à 93 % en poids du poids total de la base émulsifiante.

12. Composition cosmétique telle que définie dans la revendication 1, dans laquelle un ingrédient en phase liquide est présent en une quantité de 50 à 80 % en poids, par rapport à la composition cosmétique finale.
